# EUROPÄISCHE PATENTSCHRIFT

(11) **EP 0 759 043 B1**
(45) Veröffentlichungstag und Bekanntmachung des Hinweises auf die Patenterteilung: **02.02.2000**
(21) Anmeldenummer: 95920801.8
(22) Anmeldetag: 05.05.1995
(51) Int. Cl.: C08G 18/42, C08G 63/48

(54) **VERWENDUNG VON POLYURETHANPREPOLYMEREN ENTHALTEND REAKTIONSPRODUKTE VON POLYESTERN AUF BASIS OLEOCHEMISCHER POLYOLE ZUR HERSTELLUNG VON POLYURETHAN-SCHAUMSTOFFEN**
USE OF POLYURETHANE PREPOLYMERS CONTAINING REACTION PRODUCTS OF OLEOCHEMICAL POLYOL-BASED POLYESTERS FOR PRODUCING POLYURETHANE FOAMS
UTILISATION DE PREPOLYMERES POLYURETHANNES CONTENANT DES PRODUITS REACTIONNELS DE POLYESTERS A BASE DE POLYOLS OLEOCHIMIQUES POUR PRODUIRE DES MOUSSES DE POLYURETHANNE

(30) Priorität: 13.05.1994 DE 4416838
(43) Veröffentlichungstag der Anmeldung: 26.02.1997
(73) Patentinhaber: Cognis Deutschland GmbH, 40589 Düsseldorf (DE)
(72) Erfinder: DAUTE, Peter, D-45127 Essen (DE); KLEIN, Johann, D-40233 Düsseldorf (DE); KLUTH, Hermann, D-40235 Düsseldorf (DE); GRÜTZMACHER, Roland, D-42489 Wülfrath (DE)
(86) Internationale Anmeldenummer: EP9501705
(87) Internationale Veröffentlichungsnummer: WO9531493

(56) Entgegenhaltungen:
- EP-A- 0 125 579
- EP-A- 0 196 458
- EP-A- 0 256 355
- GB-A- 825 869
- US-A- 3 085 896
- US-A- 3 318 828
- US-A- 3 827 993

## Beschreibung

### Gebiet der Erfindung

Die Erfindung betrifft die Verwendung von Polyurethan-Prepolymeren auf Basis von Polyolen, die durch Veresterung oleochemischer Polyole mit Polycarbonsäuren hergestellt werden, zur Herstellung von Einkomponenten-Polyurethanschäumen gemäß Anspruch 1.

### Stand der Technik

Die Herstellung oleochemischer Polyole aus nachwachsenden Rohstoffen ist bekannt.

In der CA-A 850 672 wird die Ringöffnung epoxidierter Triglyceride mit Diolen und die Verwendung der Reaktionsprodukte als Polyurethanrohstoffe beschrieben.

In der US-A 4 546 120 wird die Ringöffnung epoxidierter Fettalkohole mit Diolen und die Verwendung der Reaktionsprodukte zur Herstellung von Polyurethanen beschrieben.

In der DE-A1 41 24 665 wird die mit Lithium-Ionen katalysierte Herstellung von Oligoglycerin aus Glycerin und die anschließende Umesterung mit Triglyceriden beschrieben.

Die DE-A1 41 28 649 beschreibt die mit Lithiumsalzen katalysierte Ringöffnung von Epoxiden mit Nucleophilen wie Alkoholen, ggf. verbunden mit einer anschließenden Umesterung der Reaktionsprodukte mit weiteren, nicht epoxidierten Triglyceriden. Die Reaktionsprodukte können zur Herstellung von Polyurethanschäumen verwendet werden.

Polyurethanschäume sind z.B. aus Ullmannns Enzyklopädie der technischen Chemie, 4. Auflage, Band 19, S. 317 ff, Verlag Chemie, Weinheim, bekannt.

Man kann sie in Einkomponenten- und Zweikomponenten-Polyurethanschäume einteilen.

Bei den Einkomponenten- Polyurethanschäumen handelt es sich um Mischungen aus Prepolymeren, das sind mit Isocyanat-Gruppen terminierte Reaktionsprodukte von Polyolen mit di- oder polyfunktionellen Isocyanaten, Katalysatoren zur Beschleunigung der Reaktion, Mitteln zur Senkung der Viskosität, Treibmitteln und sonstigen Hilfsstoffen, die - gegen Feuchtigkeit geschützt - in Druckgefäßen in den Handel gebracht werden. Bei Freisetzen aus den Druckgefäßen kommt es durch Verdampfen des Treibmittels zum Aufschäumen der Masse, die durch einwirkende Feuchtigkeit aus der Umgebung oder der Atmosphäre aushärtet.

Zur Herstellung der Prepolymere werden die Polyole mit einem molaren Überschuß von di- oder polyfunktionellen Isocyanaten umgesetzt.

Zweikomponenten-Polyurethanschäume werden aus einer Polyol- und einer Isocyanatkomponente hergestellt. Als Treibmittel kann Wasser dienen, das mit den NCO-Gruppen unter CO₂-Bildung abreagiert, es können aber gegebenenfalls auch zusätzlich weitere Treibmittel eingesetzt werden.
Auch die Reaktion von COOH- mit NCO-Gruppen kann, wie z.B. in der DE-A1 42 02 758 beschrieben, zur Generierung von CO₂ ausgenutzt werden.
Die molaren Mengen an Isocyanat- und mit Isocyanat reaktiven Gruppen liegen hier etwa im Bereich der Äquivalenz.

Polyurethanschäume werden beispielsweise zur Isolierung von Haushaltsgeräten, in der Bauindustrie als Dämmstoff und zum Ausschäumen von Zwischenräumen verwendet.
Darüberhinaus werden Polyurethanweichschäume in der Automobil- und Möbelindustrie eingesetzt.

Die DE-A1 33 17 194 beschreibt 1-K-Polyurethanschäume, deren Prepolymere aus Ringöffnungsprodukten epoxidierter Triglyceride mit Alkoholen und polyfunktionellen Isocyanaten hergestellt wurden.

In der DE-A1 36 26 223 werden Prepolymere zur Herstellung von Polyurethanschäumen auf Basis oleochemischer Polyole beansprucht, die durch Ringöffnung von epoxidierten Triglyceriden mit Alkoholen und anschließende partielle Umesterung erhalten werden.

Die DE-A1 42 02 758 offenbart die Verwendung von Ringöffnungsprodukten der Epoxystearinsäure mit Diolen zur Herstellung von Polyurethanschaumstoffen.

Die bisher bekannten oleochemischen Polyole erlauben die Herstellung von Polyurethanschäumen, haben aber noch einige Nachteile, z.B. daß sie wegen ihrer besonders niedrigen Viskosität zu Prepolymeren führen, die nur geringe Standfestigkeiten aufweisen. Darunter versteht man die Fähigkeit des Schaums auch in relativ breiten, senkrechten Fugen während der Härtung nicht abzulaufen. Wenn die Viskosität des Prepolymeren zu gering ist, kann es zum Auslaufen der Polyurethanmasse aus der Fuge kommen. Zu hohe Prepolymerviskositäten sind natürlich auch unerwünscht, weil sie die Aufschäumbarkeit des Prepoylmeren sowie die Austrittsrate des Schaums aus dem Gebinde beeinträchtigen.

Ein weiterer Nachteil liegt darin, daß oftmals OH-Zahl, Viskosität und Funktionalität für den jeweiligen Anwendungszweck nicht optimal aufeinander abgestimmt sind.

Die Kombinierbarkeit der Polyether- und Polyesterpolyole ist wegen ihrer oftmals eingeschränkten Mischbarkeit stark erschwert. Wegen ihres in vielen Fällen optimalen Viskositätsverhaltens sind Polyether für viele Schaumanwendungen bevorzugt. Eine Anpassung anderer erwünschter anwendungstechnischer Eigenschaften wie z.B. der schwierigen Entflammbarkeit könnte durch Abmischung mit anderen Polyolen erfolgen, wenn diese mit den Polyethern mischbar sind. Dementsprechend besteht ein Bedarf für Polyester, die mit üblichen, für Polyurethanschäume verwendbaren Polyetherpolyolen mischbar sind.

Die Aufgabe der Erfindung hat darin bestanden, Polyole auf Basis oleochemischer Polyole mit einem verbesserten Viskositätsverhalten zur Verfügung zu stellen. Außerdem sollen diese Polyole mit üblichen Polyether-Polyolen besser mischbar sein als übliche Polyester-Polyole. Weiterhin sollten OH-Zahl und Funktionalität der Polyole leicht an die jeweilige Anwendung angepaßt werden können. Eine weitere Aufgabe hat darin bestanden, durch chemische Modifikation oleochemischer Polyole mit geeigneten Dicarbonsäuren die Entflammbarkeit von Polyurethanschäumen zu erschweren.

### Beschreibung der Erfindung

Gegenstand der Erfindung ist die Verwendung von Polyurethanprepolymeren enthaltend Reaktionsprodukte von Veresterungsprodukten oleochemischer Polyole mit Polycarbonsäuren oder Polycarbonsäureanhydriden mit einem Überschuß von di- oder polyfunktionellen Isocyanaten, wobei das Äquivalenzverhältnis von NCO : OH zwischen 10:1 und 2:1 liegt, zur Herstellung feinzelliger, homogener Einkomponenten-Polyurethanschäume mit verbessertem Viskositätsverhalten.

### Polyole

Die im Rahmen der vorliegenden Erfindung einzusetzenden Polyole werden aus oleochemischen Polyolen und Polycarbonsäuren oder Anhydriden von Polycarbonsäuren durch Veresterung hergestellt.

Unter oleochemischen Polyolen versteht man Polyole auf Basis natürlicher Öle und Fette, z.B. die Reaktionsprodukte von epoxidierten Fettstoffen mit mono-, di- oder polyfunktionellen Alkoholen oder Glycerinester langkettiger Fettsäuren, die zumindest teilweise mit Hydroxylgruppen substituiert sind.

Eine bevorzugt verwendete Untergruppe dieser Verbindungen sind die Ringöffnungsprodukte epoxidierter Triglyceride, also epoxidierter Fettsäureglycerinester, bei denen die Ringöffnung unter Erhalt der Esterbindungen ausgeführt worden ist. Zur Herstellung der Ringöffnungsprodukte kann man von einer Vielzahl epoxidierter Triglyceride pflanzlichen oder tierischen Ursprungs ausgehen. So sind beispielsweise epoxidierte Triglyceride geeignet, die 2 bis 10 Gewichtsprozent Epoxidsauerstoff aufweisen. Derartige Produkte sind durch Epoxidation der Doppelbindungen aus einer Reihe von Fetten und ölen herstellbar, z.B. Rindertalg, Palmöl, Erdnußöl, Rüböl, Baumwollsaatöl, Sojaöl, Sonnenblumenöl und Leinöl. Besonders bevorzugte epoxidierte Triglyceride sind epoxidiertes Sojaöl und epoxidiertes Leinöl.

Als Alkohole für die Ringöffnung der epoxidierten Triglyceride können Methanol, Ethanol, Propanol, Isopropanol, Butanol, Hexanol, 2-Ethylhexanol, Fettalkohole mit 6 bis 22 C-Atomen, Cyclohexanol, Benzylalkohol, 1,2-Ethandiol, 1,2-Propandiol, 1,3-Propandiol, 1,4-Butandiol, 1,6-Hexandiol, Neopentylglykol, Trimethylolpropan, Glycerin, Trimethylolethan, Pentaerythrit, Sorbit, Kohlenhydrate wie Glucose sowie ethergruppenhaltige Hydroxyverbindungen wie Alkylglykole oder oligomere Glykole sowie oligomere Glycerine eingesetzt werden.
Auch die Alkoxylierungsprodukte der vorgenannten Alkohole können für die Ringöffnung eingesetzt werden.

Die Ringöffnungsreaktion epoxidierter Fettsäureester oder Triglyceride mit einem Alkohol kann gegebenenfalls von einer Umesterung mit sich selber oder anderen, nachträglich zugefügten Triglyceriden, wie zum Beispiel Palmöl, Erdnußöl, Rüböl, Baumwollsaatöl, Sojaöl, Sonnenblumenöl und Leinöl, gefolgt sein. Solche oleochemischen Polyole sind z.B. in der deutschen Patentanmeldung DE-A1 41 28 649 beschrieben.

In einer besonders bevorzugten Ausführungsform der Erfindung wird die Ringöffnungsreaktion in Gegenwart von 10 bis 1000, bevorzugt 20 bis 300 ppm an Lithium-Ionen in Form von Lithiumsalzen, z.B. Lithiumhydroxid oder Lithiumacetat, durchgeführt. Falls gewünscht, kann der Ringöffnung eine Umesterungsreaktion folgen. Da Lithium-Ionen in so geringen Mengen die Reaktion von Hydroxylgruppen mit Isocyanatgruppen nicht stören und auch die Trimerisierung von Isocyanatgruppen nicht katalysieren, können sie im Poloyol verbleiben und brauchen nicht durch aufwendige Maßnahmen abgetrennt zu werden.

Eine weitere Gruppe der oleochemischen Polyole sind Ringöffnungs- und Umesterungsprodukte von epoxidierten Fettsäureestern niederer Alkohole, also von epoxidierten Fettsäuremethyl-, -ethyl-, -propyl- oder -butylestern. Bevorzugt sind hier die Ringöffnungs- und Umesterungsprodukte mit Alkoholen der Funktionalität 2 bis 4, insbesondere die Umsetzungsprodukte mit Ethylenglykol, Propylenglykol, oligomeren Ethylenglykolen, oligomeren Propylenglykolen, Glycerin, Trimethylolpropan oder Pentaerythrit. Die Herstellung derartiger Produkte kann nach bekannten Epoxidations- und Ringöffnungsverfahren erfolgen, wobei die Umesterung während oder nach dem Ringöffnungsschritt durchgeführt werden kann. Bevorzugt sind Ringöffnungs- und Umesterungsprodukte, bei denen ein molares Verhältnis zwischen epoxidiertem Fettsäureester und dem zur Umsetzung verwendetem Alkohol von 1:0,5 bis 1:10 angewandt worden ist.

Ebenfalls zu den oleochemischen Polyolen zählen die Umsetzungsprodukte epoxidierter Fettalkohole mit C2-C8-Alkoholen der Funktionalität 1 bis 10, insbesondere 2 bis 4, im molaren Verhältnis der Epoxidgruppen zu den Hydroxylgruppen von 1:1 bis 1:10.

Im Rahmen der Erfindung ist auch die Verwendung von oleochemischen Polyolen möglich, die über die Umesterung von di- oder polyfunktionellen Alkoholen wie z.B. Trimethylolpropan, Glycerin oder dem Additionsprodukt von Ethylenoxid oder Propylenoxid an Glycerin mit Triglyceriden, wie z.B. Palmöl, Erdnußöl, Rüböl, Baumwollsaatöl, Sojaöl, Sonnenblumenöl und Leinöl, zugänglich sind.

Ebenso können Polyole verwendet werden, die nach der Lehre der DE-A1 41 24 665 durch die Umesterung von polymerisiertem Glycerin mit den vorstehend genannten Triglyceriden erhältlich sind.

Auch Ricinusöl kann als oleochemisches Polyol verwendet werden.

Die oleochemischen Polyole können Hydroxylzahlen von 50 bis 500, bevorzugt 100 bis 400 aufweisen.

### Polycarbonsäuren:

Unter Polycarbonsäuren versteht man Carbonsäuren mit 2 bis 6 Carboxylfunktionen im Molekül. Beispiele für Polycarbonsäuren mit mehr als 2 Carboxylfunktionen sind Trimellitsäure, Pyromellitsäure, Trimesinsäure, Butantricarbonsäure und Butantetracarbonsäure.
Auch die Anhydride der vorgenannten Polycarbonsäuren können verwendet werden.

Bevorzugt werden Dicarbonsäuren oder deren Anhydride verwendet.

Beispiele für Dicarbonsäuren sind Oxalsäure, Malonsäure, Bernsteinsäure, Maleinsäure, Fumarsäure, Glutarsäure, Adipinsäure, Korksäure, Sebacinsäure, 1,11-Undecandisäure, 1,12-Dodecandisäure, Phthalsäure, Isophthalsäure, Terephthalsäure, Hexahydrophthalsäure, Tetrahydrophthalsäure oder Dimerfettsäure. Bevorzugt wird Adipinsäure verwendet.

Beispiele für Dicarbonsäureanhydride sind Bernsteinsäureanhydrid, Maleinsäureanhydrid und - bevorzugt - Phthalsäureanhydrid.

In einer besonderen Ausführungsform der Erfindung wird die Veresterung zumindest teilweise mit halogensubstituierten Dicarbonsäuren oder deren Anhydriden, z.B. Tetrachlorphthalsäureanhydrid oder Tetrabromphthalsäureanhydrid, durchgeführt.

Das Äquivalent-Verhältnis von OH:COOH bei der Veresterung der Polyole mit den Polycarbonsäuren oder Polycarbonsäureanhydriden liegt im allgemeinen zwischen 20:1 und 1,5:1, bevorzugt zwischen 10:1 und 2,5:1.

Die Veresterung kann auf übliche Weise dadurch erfolgen, daß die Mischung der Polyole und Polycarbonsäuren oder Polycarbonsäureanhydride auf Temperaturen von 100 bis 250° C erhitzt und das Reaktionswasser abgetrennt wird.

Die Veresterung wird üblicherweise bis zu einer Säurezahl < 10, bevorzugt < 3, besonders bevorzugt < 1 geführt.

Die erfindungsgemäßen Polyole können mit di- oder polyfunktionellen Isocyanaten zu Prepolymeren für 1-K Polyurethanschäume umgesetzt werden. Sie können aber auch, gegebenenfalls in Abmischung mit anderen Polyolen, bevorzugt Polyether-Polyolen, als Polyolkomponente für Polyurethanschäume verwendet werden.

Neben den erfindungsgemäßen Polyolen können zur Herstellung von 1-K PUR-Schäumen übliche Polyether- oder Polyester-Polyole eingesetzt werden, wie sie z.B. in Ullmannns Enzyklopädie der technischen Chemie, 4. Auflage, Band 19, S. 304-5, Verlag Chemie, Weinheim, beschrieben sind.

### Isocyanate

Als di- oder polyfunktionelle Isocyanate können die in der Polyurethanchemie üblichen, dem Fachmann bekannten Produkte eingesetzt werden, wie sie z.B. in Ullmannns Enzyklopädie der technischen Chemie, 4. Auflage, Band 19, S. 303-4, Verlag Chemie, Weinheim, beschrieben sind.
Bevorzugt werden aromatische di- oder polyfunktionelle Isocyanate, z.B. Toluylendiisocyanat in Form der 2,4- oder 2,6-Isomeren oder deren Gemische eingesetzt, besonders bevorzugt Diphenylmethandiisocyanat und/oder die höherfunktionelle Moleküle enthaltenden technischen Qualitäten dieses Diisocyanats.

Zur Herstellung von 1-K Polyurethanschäumen werden die erfindungsgemäßen Polyole im allgemeinen mit einem molaren Überschuß an Isocyanat-Gruppen über die OH-Gruppen umgesetzt. Der Überschuß wird so gewählt, daß das Äquivalentverhältnis von NCO:OH zwischen 10:1 und 2:1, bevorzugt zwischen 6:1 und 3:1 liegt.

### Hilfsmittel

Als Treibmittel werden bevorzugt höherfluorierte Chlorkohlenwasserstoffe (HFCKW's) wie z.B. Difluorchlormethan, Difluorchlorethan, Dichlorfluorethan oder Tetrafluorethan, Fluorkohlenwasserstoffe, wie z.B. Difluorethan oder Halogenfreie Treibmittel wie Dimethylether, Propan, iso-Butan oder Pentan verwendet. Im Rahmen der Erfindung können jedoch auch FCKW-haltige Treibmittel verwendet werden.

Als weiteres übliches Treibmittel kann Wasser verwendet werden.

Für die mit Carbonsäuren getriebenen Schäume werden Carbonsäuren als Treibmittel verwendet, bevorzugt die in der DE-A1 42 02 758 genannten Carbonsäuren.

Die Reaktionsmischungen zur Herstellung von Polyurethanschäumen können noch weitere Verbindungen, wie Flammschutzmittel, z.B. die aus dem Stand der Technik bekannten Tris-(Chloralkyl-)phosphate oder -arylphosphate enthalten. Die Menge an Flammschutzmittel kann im Bereich von 8 bis 35 Gew. % bezogen auf die gesamte Mischung liegen.

Als Katalysatoren zur Beschleunigung der Reaktion können z. B. die dem Fachmann bekannten tertiären Amine, z.B. 2,2'-Dimorpholinodiethylether oder N-Methylimidazol, eingesetzt werden.

Als Stabilisator können Siloxan-Oxialkylen-Copolymere verwendet werden.

Die Mengen an Katalysator und Stabilisator können jeweils zwischen 0,001 und 3 Gew.% bezogen auf die gesamte Mischung liegen.

Die Herstellung der erfindungsgemäßen Polyolkomponenten für Polyurethanschäume kann dadurch erfolgen, daß man die Veresterungsprodukte der oleochemischen Polyole mit den Polycarbonsäuren mit dem Treibmittel, den Katalysatoren, den Stabilisatoren, den Flammschutzmitteln und gewünschtenfalls anderen Polyolen zu einer Polyolkomponente abmischt.
Die Herstellung eines Schaums erfolgt nach Vermischen der erfindungsgemäßen Polyolkomponente mit einer Isocyanatkomponente. Das Mischen kann in einem Mischungsaggregat vorgenommen werden, beispielsweise in einem Statikmischer oder einer Mischdüse.

Die Herstellung der erfindungsgemäßen Polyurethanprepolymere für Einkomponenten-Polyurethanschäume erfolgt dadurch, daß die erfindungsgemäßen Polyole mit dem di- oder polyfunktionellen Isocyanat, dem Treibmittel, dem Flammschutzmittel, dem Katalysator und ggf. dem Stabilisator gemischt und in ein Druckgefäß mit Sprühventil abgefüllt werden.

Nach dem Austreten des unter Druck stehenden Gemisches aus dem Ventil bildet sich in üblicher Weise ein Polyurethan-, schaum, der z.B. zum Ausschäumen von Fugen, zur Isolierung und dergleichen verwendet werden kann.

Die erfindungsgemäßen Polyurethanprepolymere können in Form von 1-K Polyurethanschäumen vor allem im Bausektor zur Herstellung feinzelliger, homogener Polyurethanschäume verwendet werden.

### Beispiele

Alle prozentualen Angaben in den Beispielen verstehen sich, sofern nicht anders angegeben, in Gewichtsprozent.

### Vergleichspolyole:

PPG 2020 ist ein Polypropylenglykol mit Molekulargewicht 2000.

Voranol^{(R)} EP 1900 ist ein Polyetherdiol auf Basis von Polypropylenglykol der Fa. DOW mit einer OH-Zahl von 25-30.

Voranol^{(R)} CP 3055 ist ein Polyethertriol auf Basis von Polypropylenglykol der Fa. DOW mit einer OH-Zahl von 53-59.

Vergleich 1 ist ein Polyester aus Adipinsäure, Diethylenglykol, 1,6-Hexandiol und Neopentylglykol mit einer OH-Zahl von 60.

Vergleich 2 ist ein Polyester aus Isophthalsäure, Adipinsäure, 1,2-Propylenglykol und Diethylenglykol mit einer OH-Zahl von 160.

Vergleich 3 ist ein Umesterungsprodukt aus Rüböl und Glycerin x 1 mol Propylenoxid mit einer OH-Zahl von 293.

### Beispiel 1: Veresterungsprodukt

In einem 2 l Dreihalskolben mit Rührer, Rückflußkühler mit Wasserabscheider und Stickstoffeinleitung werden 800 g Rüböl (Zusammensetzung: 50-66 % ölsäure, 18-30 % Linolsäure, 6-14 % Linolensäure, 4-10 % gesättigte Fettsäuren, Verseifungszahl = 190) und 460 g Glycerin mit 0,126 g LiOH versetzt und 6 h auf 240 °C erwärmt. Dabei findet keine Wasserabscheidung statt. Nach dem Abkühlen werden 226 g Phthalsäureanhydrid zugegeben und ca. 6 Stunden unter Abscheiden von Wasser verestert bis eine Säurezahl von 1 erreicht ist. Das Endprodukt wird noch für 1 Stunde bei 110 °C im Vakuum (ca. 10 mbar) getrocknet. Man erhält einen Polyester mit OH-Zahl 412.

### Beispiel 2: Veresterungsprodukt

In einem 4 l Dreihalskolben mit Rührer, Rückflußkühler mit Wasserabscheider und Stickstoffeinleitung werden 2028 g Rüböl und 630 g Glycerin mit 0,266 g LiOH versetzt und 6 h auf 240 °C erwärmt. Dabei findet keine Wasserabscheidung statt. Nach dem Abkühlen werden 510 g Phthalsäureanhydrid zugegeben und ca. 6 Stunden unter Abscheiden von Wasser verestert bis eine Säurezahl von 0,6 erreicht ist. Das Endprodukt wird noch für 1 Stunde bei 110 °C im Vakuum (ca. 10 mbar) getrocknet. Man erhält einen Polyester mit OH-Zahl 216.

### Beispiel 3: Veresterungsprodukt

In einem 2 l Dreihalskolben mit Rührer, Rückflußkühler mit Wasserabscheider und Stickstoffeinleitung werden 560 g Rüböl und 240 g Glycerin mit 0,08 g LiOH versetzt und 6 h auf 240 °C erwärmt. Dabei findet keine Wasserabscheidung statt. Nach dem Abkühlen werden 200 g Adipinsäure zugegeben und ca. 6 Stunden unter Abscheiden von Wasser verestert bis eine Säurezahl von 0,2 erreicht ist. Das Endprodukt wird noch für 1 Stunde bei 110 °C im Vakuum (ca. 10 mbar) getrocknet. Man erhält einen Polyester mit OH-Zahl 223.

### Beispiel 4: Veresterungsprodukt

In einem 2 l Dreihalskolben mit Rührer, Rückflußkühler mit Wasserabscheider und Stickstoffeinleitung werden 676 g Rüböl und 210 g Glycerin mit 0,09 g LiOH versetzt und 6 h auf 240 °C erwärmt. Dabei findet keine Wasserabscheidung statt. Nach dem Abkühlen werden 96 g Phthalsäureanhydrid und 232 g Tetrabromphthalsäureanhydrid zugegeben und ca. 6 Stunden unter Abscheiden von Wasser verestert bis eine Säurezahl von 0,7 erreicht ist. Das Endprodukt wird noch für 1 Stunde bei 110 °C im Vakuum (ca. 10 mbar) getrocknet. Man erhält einen Polyester mit OH-Zahl 112.

### Verträglichkeitsprüfung:

Zur Prüfung der Verträglichkeit wurden verschiedene Polyetherpolyole mit den erfindungsgemäßen Veresterungsprodukten und zum Vergleich mit üblichen Polyestern im Verhältnis 1:1 gemischt. Nach 24 Stunden wurden die Mischungen beurteilt.
Die Ergebnisse sind in Tabelle 1 festgehalten.

**Tabelle 1:**

| Verträglichkeitsprüfung | | | |
|---|---|---|---|
| | | Polyether | |
| Polyester | PPG 2020 | Voranol EP 1900 | Voranol CP 3055 |
| | | | |
| Bsp 1 | trübe | blank | trübe |
| Bsp 3 | blank | blank | blank |
| Vergl 1 | separiert | separiert | separiert |
| Vergl 2 | separiert | trübe | separiert |

Aus dem Versuch geht die verbesserte Mischbarkeit der erfindungsgemäßen Polyester mit Polyethern eindeutig hervor.

### Prepolymer-Viskositäten:

Aus Desmodur^{(R)} VKS und einem Polyol wurden Prepolymere hergestellt, wobei das Verhältnis von NCO:OH = 4:1 gewählt wurde und die Reaktion bei 50 °C bis zu dem NCO-Gehalt geführt wurde, bei dem alle OH-Gruppen abreagiert sind.

Die Viskosität der Prepolymeren wurde mit dem Brookfield-Viskosimeter bei 40 °C mit geeigneten Spindeln und Umdrehungszahlen gemessen.

Die Werte sind in Tabelle 2 angegeben.

**Tabelle 2:**

| Prepolymer-Viskositäten | |
|---|---|
| Polyol | Viskosität [mPa·s] |
| | |
| Bsp 2 | 22.000 |
| Vergleich 1 | pastös, nicht meßbar |
| Vergleich 3 | 6.100 |

### Beispiel 5:

Herstellung eines mit Carbonsäure getriebenen Polyurethanschaums

### Rezeptur:

30,0 g Polyol nach Beispiel 1
43,0 g Desmodur^{(R)} VKS
0,5 g N-Methylimidazol
20,0 g ölsäure, Säurezahl 200
0,5 g Tegostab B 8404

Die Substanzen werden bei Raumtemperatur vermischt. Nach einer Startzeit von 30 Sekunden beginnt die Schaumreaktion, die zu einem Schaum mit einer Rohdichte von 49 g/l führt.

### Beispiel 6:

### Rezepturen für Einkomponenten-Polyurethanschäume

Es wurden drei Polyol-Komponenten nach folgender Rezeptur hergestellt.

| Polyol nach | A [g] | B [g] | C [g] |
|---|---|---|---|
| Bsp 2 | 48,3 | - | - |
| Bsp 4 | - | 53,0 | - |
| Bsp 3 | - | - | 47,8 |
| Flammschutzmittel | 47,2 | 42,5 | 47,6 |
| DABCO DC 190 | 3,6 | 3,6 | 3,6 |
| DMDEE | 0,9 | 0,9 | 0,9 |

Das Flammschutzmittel ist Tris(monochlorisopropyl)phosphat. DABCO^{(R)} DC 190 ist ein Silicontensid der Firma Air Products. DMDEE ist 2,2'-Dimorpholinodiethylether.

Aus den Polyolkomponenten A, B und C wurden Prepolymere für Einkomponenten-Polyurethanschäume hergestellt.

| Beispiel 6 | a [g] | b [g] | c [g] | d [g] | e [g] | f [g] | g [g] |
|---|---|---|---|---|---|---|---|
| Komp. A | 35,5 | - | 35,5 | - | - | - | - |
| Komp. B | - | 39,4 | - | 39,4 | - | - | - |
| Komp. C | - | - | - | - | 35,2 | 35,2 | 35,2 |
| MDI 1 | - | - | 44,5 | 40,6 | 44,8 | - | - |
| MDI 2 | 44,5 | 40,6 | - | - | - | 44,8 | - |
| MDI 3 | - | - | - | - | - | - | 44,8 |
| Treibmittel | 20,0 | 20,0 | 20,0 | 20,0 | 20,0 | 20,0 | 20,0 |

MDI 1 ist Caradate^{(R)} 30, ein Diphenylmethandiisocyanat mit einer Funktionalität von ca. 2,7 der Firma Shell.

MDI 2 ist Suprasec^{(R)} VM 50, ein Diphenylmethandiisocyanat mit einer Funktionalität von ca. 2,5 der Firma ICI.

MDI 3 ist Isonate^{(R)} 30, ein Diphenylmethandiisocyanat mit einer Funktionalität von ca. 2,4 der Firma Dow.

Das Treibmittel ist eine Mischung aus 8 Gewichtsteilen Tetrafluorethan und 2 Gewichtsteilen Dimethylether.

### Anwendungstechnische Prüfung:

Das 1-K-Polyurethangemisch wurde aus Druckdosen in 40 mm breite und 400 mm lange Fugen aus einem Holzwerkstoff gesprüht. Die Fuge war mit befeuchtetem Karton ausgekleidet. Die Mischungen ergaben homogene Schäume.

## Patentansprüche

1. Verwendung von Polyurethanprepolymeren, enthaltend Reaktionsprodukte von Veresterungsprodukten oleochemischer Polyole mit Polycarbonsäuren oder - anhydriden mit einem Überschuß von di- oder polyfunktionellen Isocyanaten, wobei das Äquivalenzverhältnis von NCO : OH zwischen 10 : 1 und 2 : 1 liegt, zur Herstellung feinzelliger, homogener Einkomponenten-Polyurethanschäume mit verbessertem Viskositätsverhalten.

2. Verwendung nach Anspruch 1, dadurch gekennzeichnet, daß als Polycarbonsäuren Dicarbonsäuren oder deren Anhydride eingesetzt werden.

3. Verwendung nach Anspruch 1, dadurch gekennzeichnet, daß als Dicarbonsäuren oder deren Anhydriden halogenierte Dicarbonsäuren eingesetzt werden.

4. Verwendung nach einem der Ansprüche 1 bis 3, dadurch gekennzeichnet, daß als oleochemische Polyole Ringöffnungsprodukte von epoxydierten Triglyceriden mit Alkoholen und gegebenenfalls deren Umesterungsprodukten eingesetzt werden.

5. Verwendung nach einem der Ansprüche 1 bis 4, dadurch gekennzeichnet, daß als oleochemische Polyole Umesterungsprodukte von di- oder polyfunktionellen Alkoholen mit Triglyceriden eingesetzt werden.

6. Verwendung nach einem der Ansprüche 1 bis 5, dadurch gekennzeichnet, daß als oleochemische Polyole solche mit OH-Zahlen von 50 bis 500 eingesetzt werden.

## Claims

1. The use of polyurethane prepolymers containing reaction products of esterification products of oleochemical polyols with polycarboxylic acids or anhydrides with an excess of difunctional or polyfunctional isocyanates, the equivalence ratio of NCO to OH being between 10:1 and 2:1, for the production of fine-cell, homogeneous one-component polyurethane foams with improved viscosity behaviour.

2. The use claimed in claim 1, characterized in that dicarboxylic acids or their anhydrides are used as the polycarboxylic acids.

3. The use claimed in claim 1, characterized in that halogenated dicarboxylic acids are used as the dicarboxylic acids or their anhydrides.

4. The use claimed in any of claims 1 to 3, characterized in that ring opening products of epoxidized triglycerides with alcohols and optionally transesterification products thereof are used as the oleochemical polyols.

5. The use claimed in any of claims 1 to 4, characterized in that transesterification products of difunctional or polyfunctional alcohols with triglycerides are used as the oleochemical polyols.

6. The use claimed in any of claims 1 to 4, characterized in that the oleochemical polyols used have OH values of 50 to 500.

## Revendications

1. Utilisation de prépolymères de polyuréthane renfermant des produits réactionnels de produits d'estérification de polyols oléochimiques avec des acides ou des anhydrides polycarboxyliques avec un excès d'isocyanates di- ou polyfonctionnels, le rapport des équivalents NCO:OH étant compris entre 10:1 et 2:1, pour la production de mousses de polyuréthane à un composant homogènes, à fines cellules et à comportement de viscosité amélioré.

2. Utilisation selon la revendication 1, caractérisée en ce que l'on met en oeuvre comme acides polycarboxyliques, des acides dicarboxyliques ou les anhydrides de ceux-ci.

3. Utilisation selon la revendication 1, caractérisée en ce que l'on met en oeuvre comme acides dicarboxyliques ou anhydrides de ceux-ci, des acides dicarboxyliques halogénés.

4. Utilisation selon une des revendications 1 à 3, caractérisée en ce que l'on met en oeuvre comme polyols oléochimiques, des produits d'ouverture de cycles de triglycérides époxydés par des alcools et, le cas échéant, les produits de transestérification de ceux-ci.

5. Utilisation selon une des revendications 1 à 4, caractérisée en ce que l'on met en oeuvre comme polyols oléochimiques, des produits de transestérification d'alcools di- ou polyfonctionnels avec des triglycérides.

6. Utilisation selon une des revendications 1 à 5 caractérisée en ce que l'on met en oeuvre comme polyols oléochimiques, ceux présentant un indice d'hydroxyle de 50 à 500.
